# EUROPEAN PATENT SPECIFICATION

(11) **EP 0 328 403 B1**
(45) Date of publication and mention of the grant of the patent: **18.06.1997**
(21) Application number: 89301288.0
(22) Date of filing: 10.02.1989
(51) Int. Cl.: C07K 14/16, G01N 33/569, A61K 38/16, A61K 39/21

(54) **Synthetic peptides related to the HIV-GP120-env-protein, and their use**
Synthetische vom HIV-GP120-env-Protein abgeleitete Peptide und ihre Anwendung
Peptides synthétiques relatifs à la protéine HIV-GP120-env. et leur application

(30) Priority: 12.02.1988 US 155321
(43) Date of publication of application: 16.08.1989
(73) Proprietor: United Biomedical Inc., Lake Success New York 11042 (US)
(72) Inventor: Wang, Chang Yi, Great Neck, N.Y. 11021 (US)
(74) Representative: Froud, Clive

(56) References cited:
- EP-A- 0 273 716
- EP-A- 0 279 688
- EP-A- 0 298 633
- EP-A- 0 306 219
- EP-A- 0 311 219
- WO-A-86/02383
- WO-A-87/07616
- WO-A-88/00471
- WO-A-88/09181
- FR-A- 2 603 107
- CHEMICAL ABSTRACTS, vol. 106, no. 3, 19 January 1987, Columbus, OH (US); p. 469, no. 16942b
- CHEMICAL ABSTRACTS, vol. 106, March 1987, Columbus, OH (US); S. MODROW et al., p. 506. no. 100556m
- PROCEEDINGS OF THE NATL. ACADEMY OF SCIENCES USA, vol. 83, September 1986, Washington, DC (US); W.G. ROBEY et al., pp. 7023-7027

## Description

This invention relates to a method using synthetic peptides as solid phase immunoadsorbents for the detection of antibodies to Human Immunodeficiency Virus (HIV) gp120, and, in particular, antibodies having HIV neutralizing capabilities. The amino acid sequences of the peptides correspond to segments of the external envelope protein gp120 of HIV. These peptides have been found to be highly immunogenic, and are reactive with antibodies in sera of patients with AIDS, ARC or HIV infected individuals. They can also be used to elicit the production of neutralizing antibodies to HIV. More specifically, the present invention is directed to the use of a synthetic peptide containing thirty-three amino acids in a prescribed sequence, their analogues and mixtures, corresponding to the HIV-gp120 external protein for the detection of antibodies to HIV-gp120. It is particularly useful for the detection of antibodies having HIV neutralizing capabilities. The detection method includes an enzyme linked immunoassay and other forms of immunoassay procedures. The present invention also relates to a method for generating high titer antibodies to HIV gp120 protein in healthy mammals, including humans, by the use of the synthetic peptides, their analogues or mixtures in either a conjugated or a polymeric form as a key component in a synthetic vaccine for the prevention of AIDS.

Since the human immunodeficiency virus (HIV) started its spread through the human population, the AIDS epidemic has steadily increased worldwide for the lack of any therapeutic or preventive means for intervention. The unique pathogenicity and the variability of HIV have raised new challenges in the design, testing and evaluation of therapeutics and vaccines for HIV. It appears, for the moment, that the development of an effective method for the detection of HIV infection and an effective vaccine remain the only means by which the disease is to be controlled and eradicated.

An ideal vaccine against HIV infection should be highly immunogenic, induce both T and B cell virus-specific immunity, and be free of irrelevant carrier proteins. Although traditional approaches using whole virion or virion subunits can generally achieve this goal, practical consideration, such as the safety and availability of native antigen, have led many to consider other more highly engineered vaccine constructs for AIDS.

Studies on the feasibility of a virion subunit vaccine to protect against viral infection have mainly focused on the envelope protein of the HIV, the precursor gp160 protein, and its derived external protein, gp120, and transmembrane protein, gp41.

The transmembrane protein gp41 of HIV has been found to be highly antigenic in that upon infection, all individuals develop antibodies to gp41 which have been detected by recombinant proteins containing segments of gp41 (1) or by synthetic peptides covering a well defined region of this protein (2).

It is well-documented that HIV infected subjects make antibodies to gp120 (3). However, the immunogenic sites and their corresponding amino acid sequences on this molecule have not yet been identified.

A recent article reported the status of the intense efforts directed to the finding of a suitable candidate for a vaccine (4). These efforts are mainly directed to the use of the external portion of the HIV envelope glycoprotein, gp120. Native gp120 was purified from HIV producing cell lines but gave very low yields (3). Moreover, although native gp120 showed toxic effect on CD4 positive cells, it only elicited moderate neutralizing antibody titers and failed to elicit antibody-dependent cell mediated cytotoxicity in the studies using chimpanzee hosts (3).

Recombinant proteins that contain the whole envelope protein gp160, the external envelope protein gp120, or portions of gp120 have been produced in insect cells (5), mammalian cells (6), yeast cells (7), and in Escherichia Coli (8). Purified gp120 has also been shown to bind the cell receptor CD4 (9,10), and to elicit neutralizing antibodies from immunized animals (11). Reports employing synthetic peptides representing small segments of gp120 have revealed various regions to be immunoreactive, these include two modest antigenic regions as being reactive to sera from a few HIV infected patients (12,13), and two regions as representative of helper T cell antigenic sites (14), one region which inhibits HIV receptor binding and T cell infectivity (15), and one region which elicits marginal neutralizing antibodies (16,17).

Pert et al. reported an 8mer peptide, named peptide T, as having the ability to inhibit the binding of I-125 labelled HIV gp120 to T4 antigen in brain membranes and block HIV infection of human T cells in vitro (15). Cease et al. reported the identification of an antigenic site of HIV-gp120, represented by a 16mer peptide, named peptide T1, that elicits specifically T cell immunity. However, no results on the peptide's immunoreactivity with serum antibodies derived from HIV infected individuals (i.e. 3 cell immunity) were reported (14). Palker et al. reported that a 15mer peptide, Sp-22, derived from a conserved region at the carboxyl terminus of HIV-gp120, showed moderate immunoreactivity with antibodies of HIV infected individuals. However, these SP-22 peptide reactive anti-gp120 antibodies have clearly been shown to be non-neutralizing (12). Contrary to Palker et al.'s report, Chanh et al. described that a synthetic 30mer peptide, having an overlapping sequence with Palker et al.'s SP-22, showed weak immunogenicity in eliciting neutralizing antibodies to gp120 (16,17). Similarly, Cosand described a peptide conjugate designated as peptide 36-BSA as having moderate antigenic properties with serum antibodies from a few HIV infected individuals (13). Cosand's peptide-36, a 24mer, has an overlapping amino acid sequence with Palker's SP-22 peptide and Chanh's 30mer peptide.

These peptides, their amino acids sequences and reported immunoreactivities are summarized in Table I.

There may also be mentioned WO 86/02383, which relates to envelope antigens of lymphadenopathy associated virus and their applications, and WO 88/00471, which concerns a composition of matter and method of immunizing against viral causative agents of AIDS and ARC.

Our previous attempts in the identification and characterization of highly antigenic epitopes on the gp41 and p24 HIV proteins have made possible the development of efficient HIV antibody screening and diagnostic method using synthetic peptides, instead of the virus itself, as the solid immunoadsorbent (2). A similar task in mapping the reactivity of neutralizing antibodies to epitopes on human retroviral protein, in particular the HIV gp120 protein, remains another challenge enroute to the design and development of synthetic vaccines for inducing high neutralizing antibody titers and specific cellular immune response to successfully prevent the spread of AIDS.

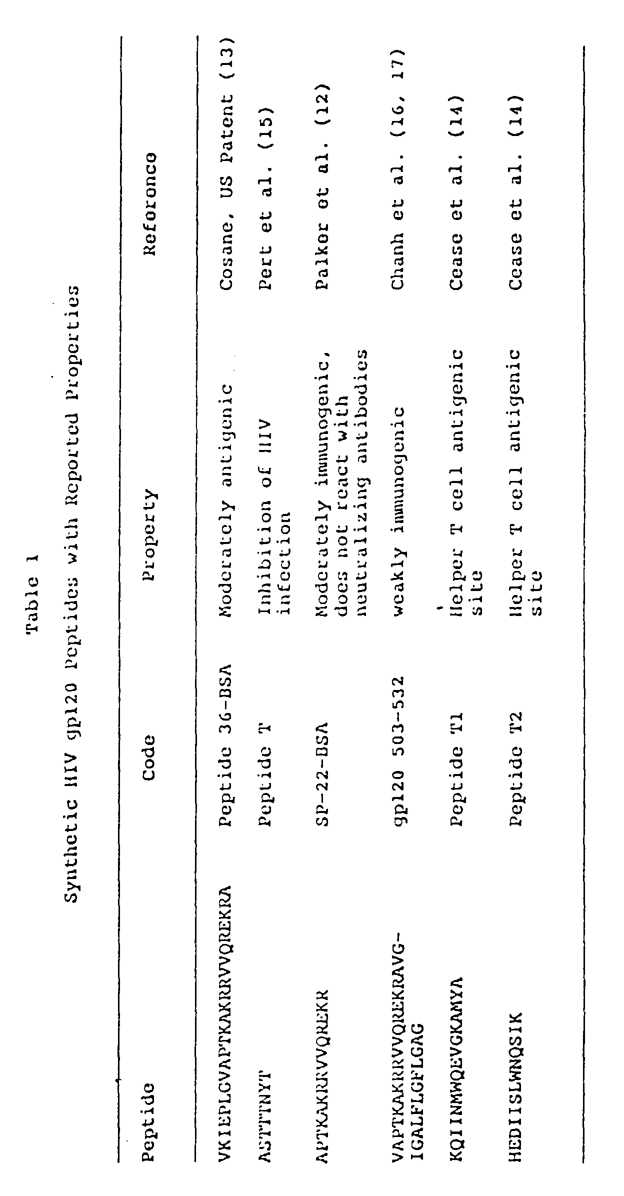

In this application, standard single letter symbols for the amino acids are used through out. These represent the amino acids as follows:

| Amino Acid | Single Letter Symbols |
|---|---|
| Alanine | A |
| Arginine | R |
| Aspartic Acid | D |
| Asparagine | N |
| Cysteine | C |
| Glycine | G |
| Glutamic Acid | E |
| Glutamine | Q |
| Histidine | H |
| Isoleucine | I |
| Leucine | L |
| Lysine | K |
| Methionine | M |
| Phenylalanine | F |
| Proline | P |
| Serine | S |
| Threonine | T |
| Tryptophan | W |
| Tyrosine | Y |
| Valine | V |

### References

1. Chang, T.W. et al. Biotechnology, 3, 905-909 (1985)
2. Wang, J.J.G. et al. Proc. Natl. Acad. Sci. USA, 83, 9709-9713 (1986)
3. Matthews, T.J. et al. Proc. Natl. Acad. Sci. USA, 83, 9709-9713 (1986)
4. Homsy, J. et al. Immunology Today, 8, 193-196 (1987)
5. Rusche, J.R. et al. Proc. Natl. Acad. Sci. USA, 84, 6924-6928 (1986)
6. Lasky, L.A. et al. Science, 233, 209-212 (1986)
7. Barr, P.J. et al., Vaccine, 5, 90 (1987)
8. Putney, S.D. et al. Science, 234, 1392-1395 (1986)
9. Dalgleish, A.G. et al. Nature (London), 312, 763-768 (1984)
10. McDougal, J.S. et al. J. Immunol., 135, 3151-3157 (1985)
11. Robey, W.G. et al. Proc. Natl. Acad. Sci. USA, 83, 7023-7027
12. Palker, T.J. et al. Proc. Natl. Acad. Sci. USA, 84, 2479-2483 (1987)
13. Cosand, W. US PATENT No. 4,629,783
14. Cease, K.B. et al. Proc. Natl. Acad. Sci. USA. 84, 4249-4253 (1987)
15. Pert, C.B. et al. Proc. Natl. Acad. Sci. USA 83, 9254-9258 (1986)
16. Chanh, T.C. et al. EMBO, 5, 3065-3071 (1986)
17. Chanh, T.C. et al. Eur. J. Immunol., 16, 1465-1468 (1986)
18. Merrifield, R.B. J.A.C.S., 85, 2149-2154 (1963)
19. Prince, A.M. et al. J. of Infectious Diseases, 156, No. 2, 268-272 (1987)

The present invention provides a peptide composition characterised in that it exhibits specific immunoreactivity to antibodies to HIV and elicits the production of neutralizing antibodies to HIV, which is selected from:
(i) QSVEINCRTRPNNNTRKSIRIQRGPGRAFVTIGK (Peptide 127);
(ii) an analogue thereof having an amino acid sequence derived from a strain/isolate of HIV in a region corresponding to the peptide;
(iii) a conjugate thereof with a carrier;
(iv) a polymer thereof; and
(v) a mixture of the peptide with an analogue, conjugate or polymer thereof.

For example, the present peptide composition may comprise a conjugate of the peptide and a protein having a molecular weight of at least 5,000, such as bovine serum albumin.

In one preferred embodiment, the present peptide composition may comprise:

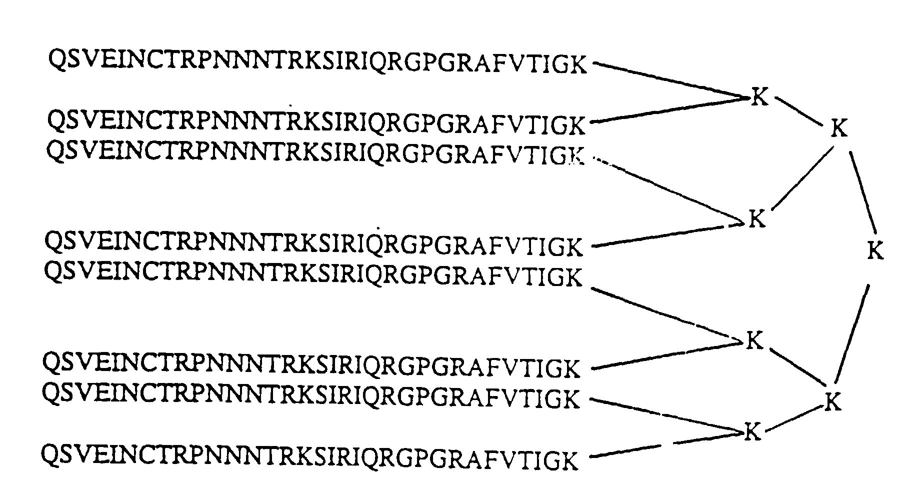

The present invention also provides a method for detecting the presence of antibodies to HIV and the presence of neutralizing antibodies to HIV-gp120 in an immunoassay characterised in that it comprises using as the immunosorbent a peptide composition as defined above.

In another embodiment, the present invention provides an analogue of peptide-127 characterised in that it is selected from:

(i) TSVEINCTRPNNNTRKRIRIQRGPGRAFVTIGK (Peptide-127HXB2)
(ii) ESVAINCTRPNNNTRKSIRYQRGPGRAFHTTGR (Peptide-127SP2)
(iii) ESVEINCTRVYRNLSKRIQRGPGRAFRTREI (Peptide-127WMJ2)
(iv) KSVEINCTRPNNNTKKGIAIGPGRTLYAREK (Peptide-127NY5)
(v) ASVQINCTRPNNNTRKSITKGPGRVIYATGQ (Peptide-127RF)
(vi) ETVTINCTRPGNNTRRGIHFGPGQALYTTGI (Peptide-127MAL)
(vii) KYYNLSLHKRPGNKTVKQIMLMSHRVFHSHYQ (Peptide-127HIV-2).

The present invention further relates to neutralising antibodies for AIDS virus characterised in that they are elicited by the use of a peptide composition as defined above.

Another such synthetic peptide, Peptide 126: CRIKQIINMWQEVGKAMYAPPISGQIRC is described in EP-A- 328403. Although peptide 126 forms no part of the present invention, that being as indicated above, some references thereto will be made herein for convenience and completeness.

The synthetic peptides of the present invention are useful for the detection of antibodies to HIV in physiological fluids and in particular are useful for the detection of neutralizing antibodies to HIV-gp120 in physiological fluids.

The peptides of the present invention are also useful as key components in vaccines for the immunization of animals including humans to elicit the production of antibodies to HIV.

Referring to the accompanying illustrative drawings:

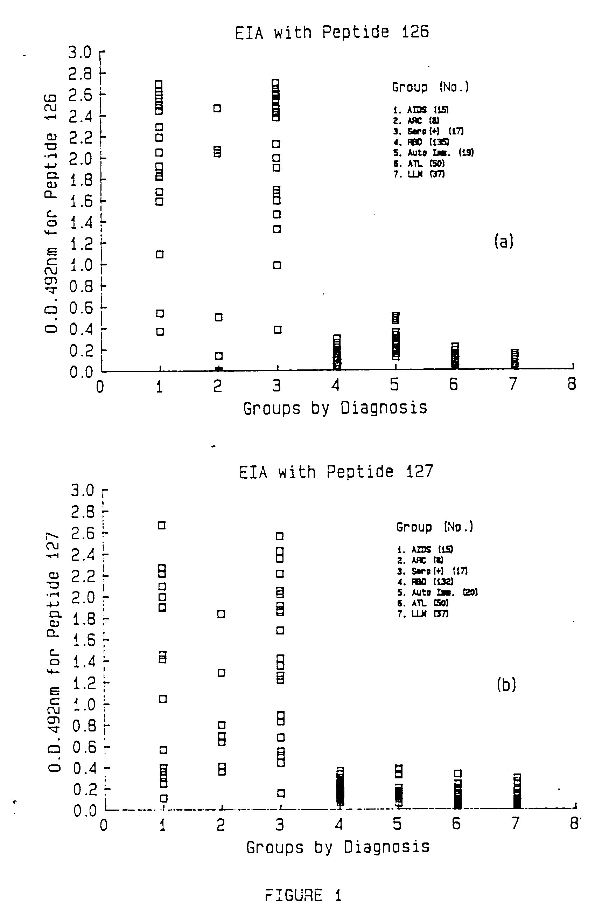

Figs. 1a and 1b respectively show the results of EIA assays using Peptide 126 and Peptide 127 as the solid phase immunoadsorbent using sera from patients diagnosed to have AIDS, ARC, persons who are known to be seropositive, lymphoid-leukemia malignancies (LLM), autoimmune diseases, adult T-cell leukemia (ATL) and from random blood donors.

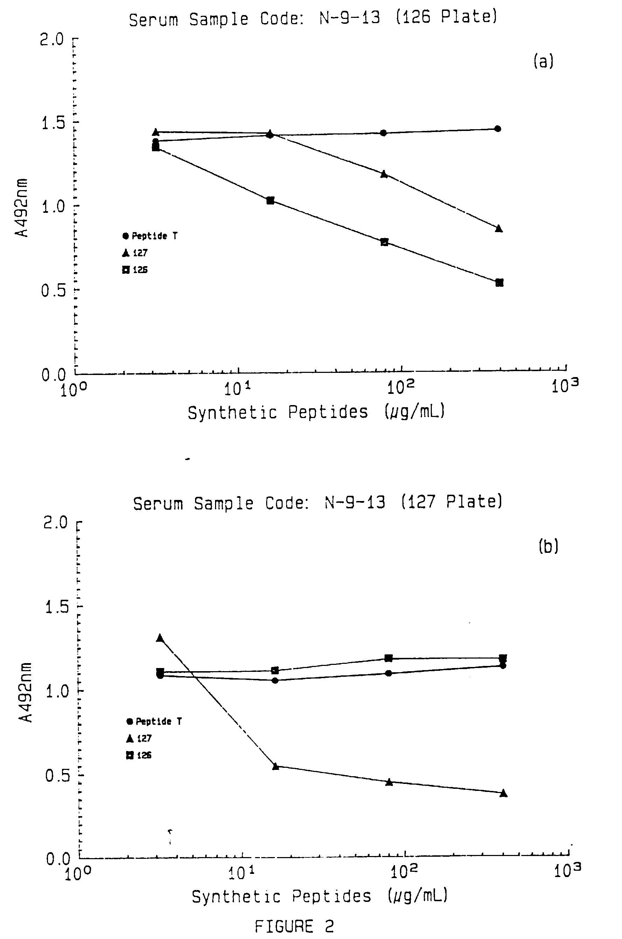

Fig. 2a and 2b show the specificity of the immunoreactivities of Peptide 126 and Peptide 127 with HIV serum antibodies. An increased amount of Peptide 126, Peptide 127 or Peptide T is pre-incubated with serum antibodies from a representative sample (N-19-13) followed by EIA using either Peptide 126 (Fig. 2a) or Peptide 127 (Fig. 2b) as solid phase immunoadsorbent.

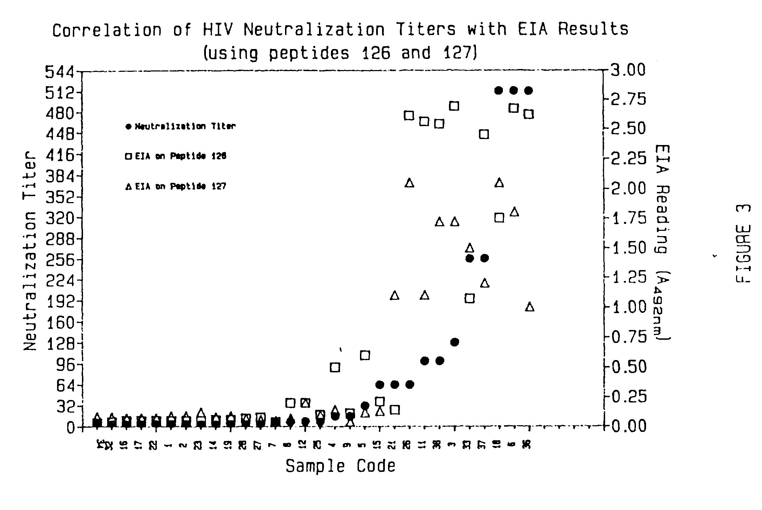

Fig. 3 shows the correlation of the results obtained by EIA using Peptide 126 and Peptide 127 and the neutralizing antibodies titer of 30 serum samples.

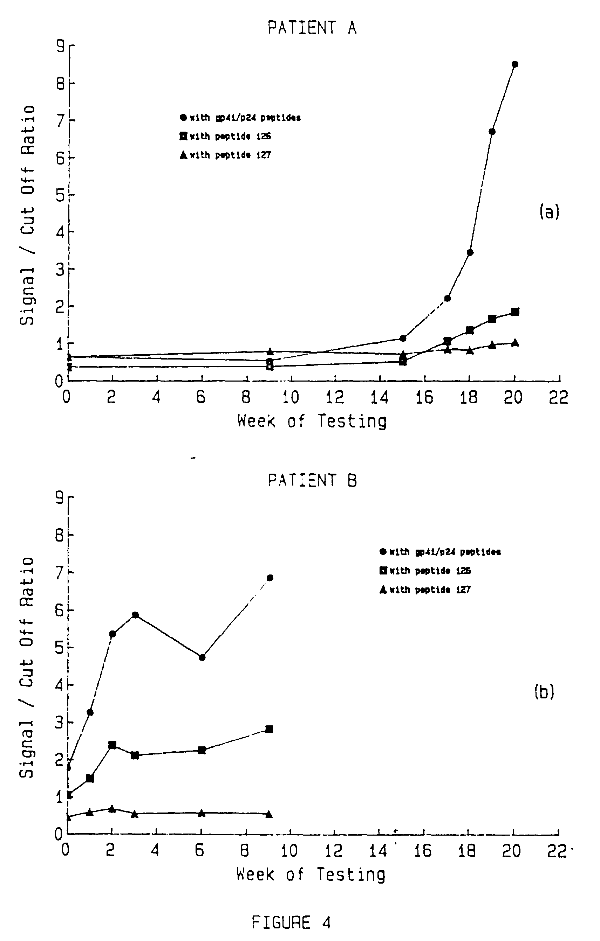

Fig. 4a and 4b shows the respective immunoreactivity of Peptide 126 and Peptide 127 with a series of serum specimens derived from Patients A and B who underwent seroconversion. At the time of serum collection when the signal/cutoff ratio derived from the gp41/p24 peptide mixture based EIA become larger than 1, HIV infection in the respective patient is detected.

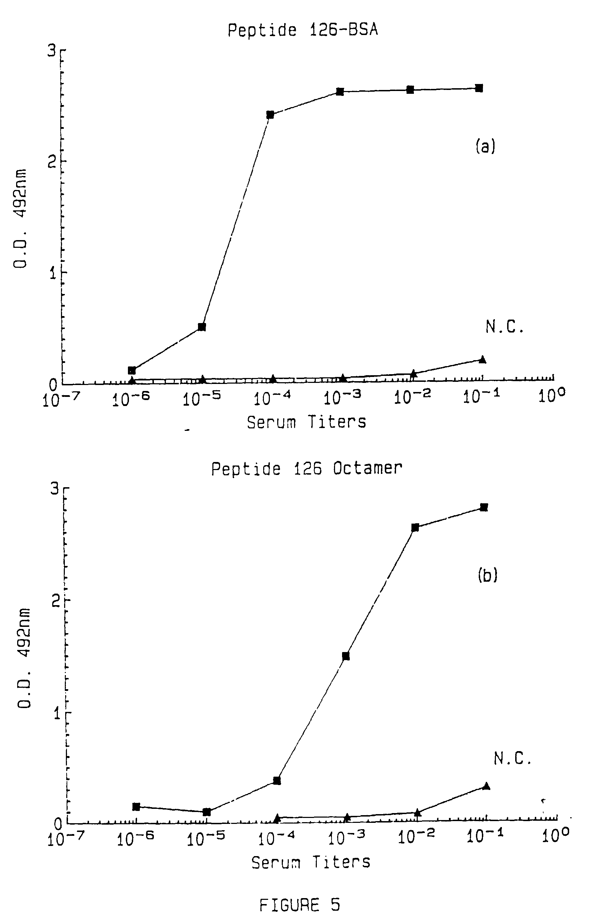

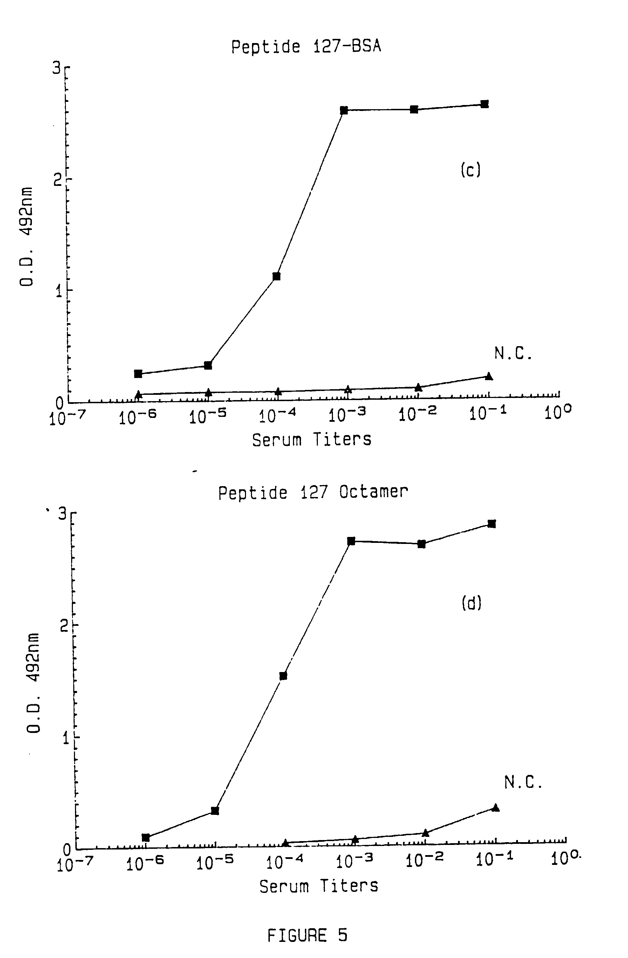

Figs. 5a, 5b, 5c and 5d show the guinea pig serum antibody titers after initial immunization and one booster shot using (i) Peptide 126-BSA (Fig. 5a), (ii) Peptide 127-BSA (Fig. 5c), (iii) Peptide 126 octamer (Fig. 5b) and (iv) Peptide 127 octamer (Fig. 5d).

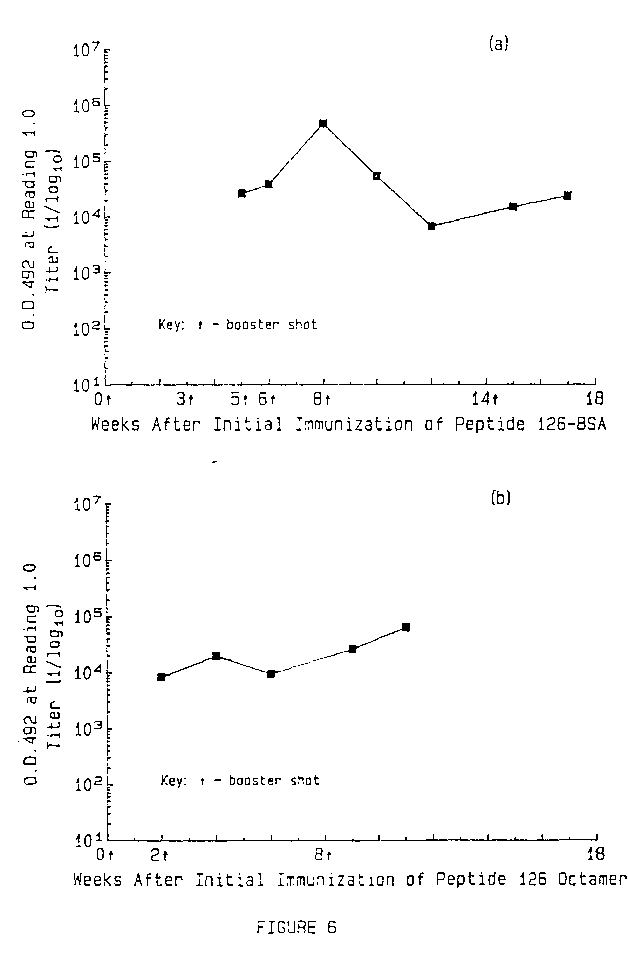

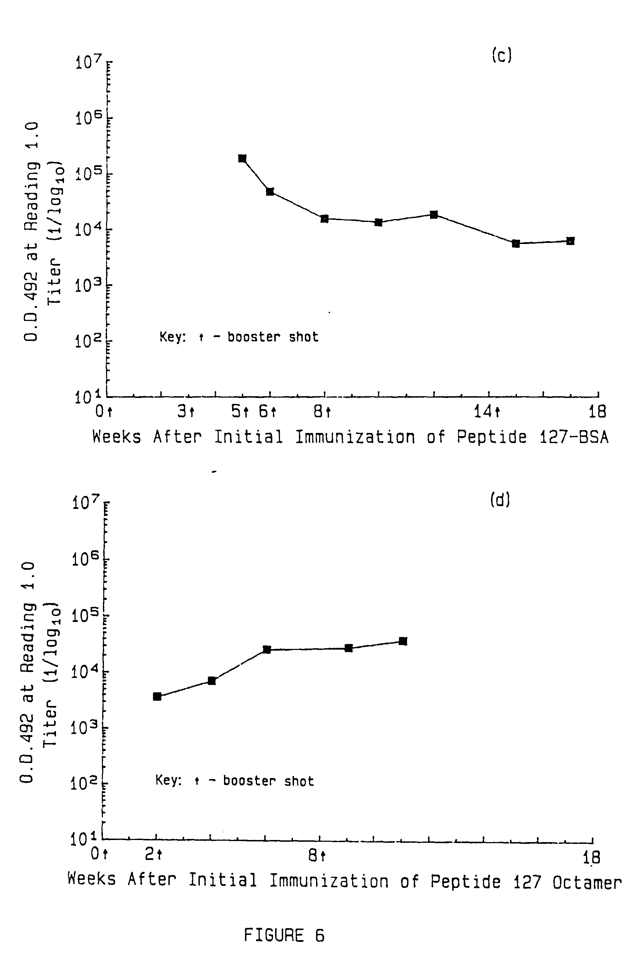

Fig. 6a, 6b, 6c and 6d show that sustained high titers of serum antibodies were produced by immunization with (i) Peptide 126-BSA (Fig. 6a), (ii) Peptide 127-BSA (Fig. 6c), (iii) Peptide 126 octamer (Fig. 6b), and (iv) Peptide 127 octamer (Fig. 6d).

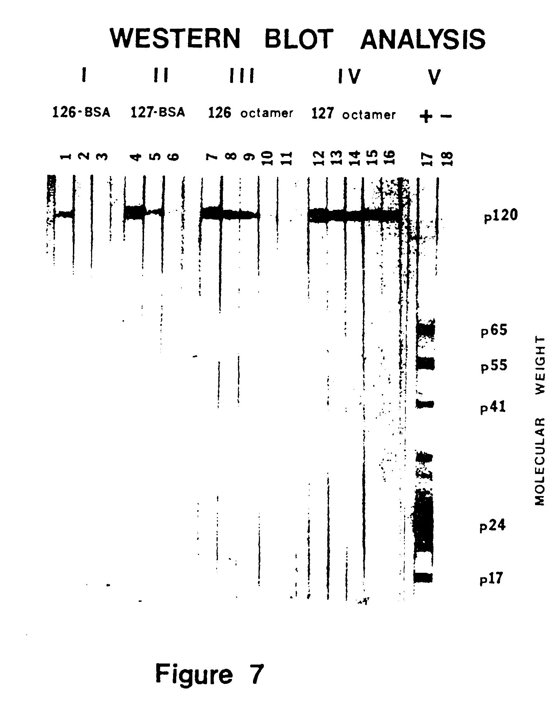

Fig. 7 shows the results of Western Blot Analysis which demonstrates the production of high level of specific anti-gp120 antibodies by immunizing guinea pigs with (I) Peptide 126-BSA at dilutions of 1:200, 1:1,000, and 1:2,000 (lanes 1-3), (II) Peptide 127-BSA at dilutions of 1:200, 1:1,000 and 1:2,000 (lanes 4-6), (III) Peptide 126 octamer at dilutions of 1:200, 1:1,000, 1:2,000, 1:10,000 and 1:20,000 (lanes 7-11), and (IV) Peptide 127 octamer at dilutions of 1:200, 1:1,000, 1:2,000, 1:10,000 and 1:20,000 (lanes 12-16), as compared with a serum sample obtained from a guinea pig immunized with (V) deactivated whole viral lysate at dilutions of 1:100 (lane 17) and a serum sample obtained from a control unimmunized guinea pig at dilution of 1:100 (lane 18).

The present invention provides novel peptides which immunologically mimic HIV gp120 external envelope protein encoded by the env region of the viral genome.

The present peptides have amino acid sequences which correspond to QSVEINCTRPNNNTRKSIRIQRGPGRAFVTIGK (Peptide 127) encoded within the bp6669 to bp6767 region of gp120. The peptides are coded herein as Peptide 127 for ease of identification.

The amino acid sequence of Peptide 127 is taken from a highly variable region in gp120 as shown in Table II. The novel peptides show a high level of immunoreactivity with antibodies to HIV and are useful in a highly sensitive and specific enzyme immunoassay (EIA) for the detection of antibodies to HIV in serum. The EIA results obtained by using. Peptide 127 also show a high degree of correlation with antibody neutralization titer and is useful as a rapid and simple method for detection of HIV neutralizing antibodies in sera.

The present peptides may be modified by introducing conservative substitutions or non-conservative substitutions, deletions or additions of amino acids to more effectively mimic the differing epitopes of the different retroviral strains as long as the specific immunoreactivity to antibodies to HIV and antibodies to HIV gp120 is preserved.

It is contemplated that the peptides and the analogues of interest will include about 15 amino acids, usually fewer than 50, more usually fewer than about 40 amino acids included within a sequence coded for by the HIV gp120 and corresponds to the following sequence : QSVEINCTRPNNNTRKSIRIQRGPGRAFVTIGK (Peptide 127) The peptide segments should be as small as possible, while still maintaining substantially all of the sensitivity of the larger peptide. In some instances it may be desirabl'e to join two or more peptides which are non-overlapping into a longer peptide. The peptides may also be used as individual peptides,

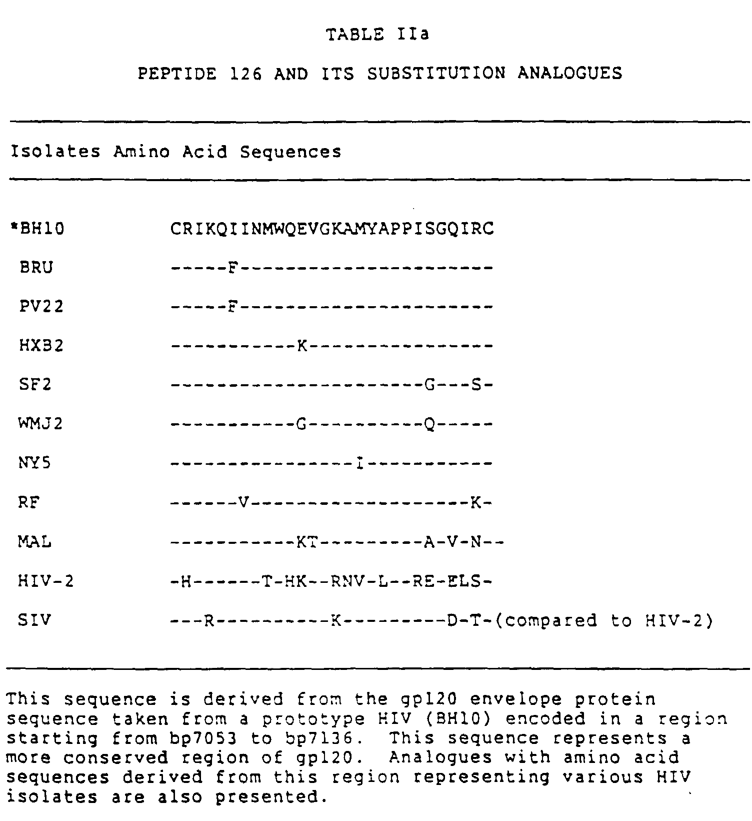

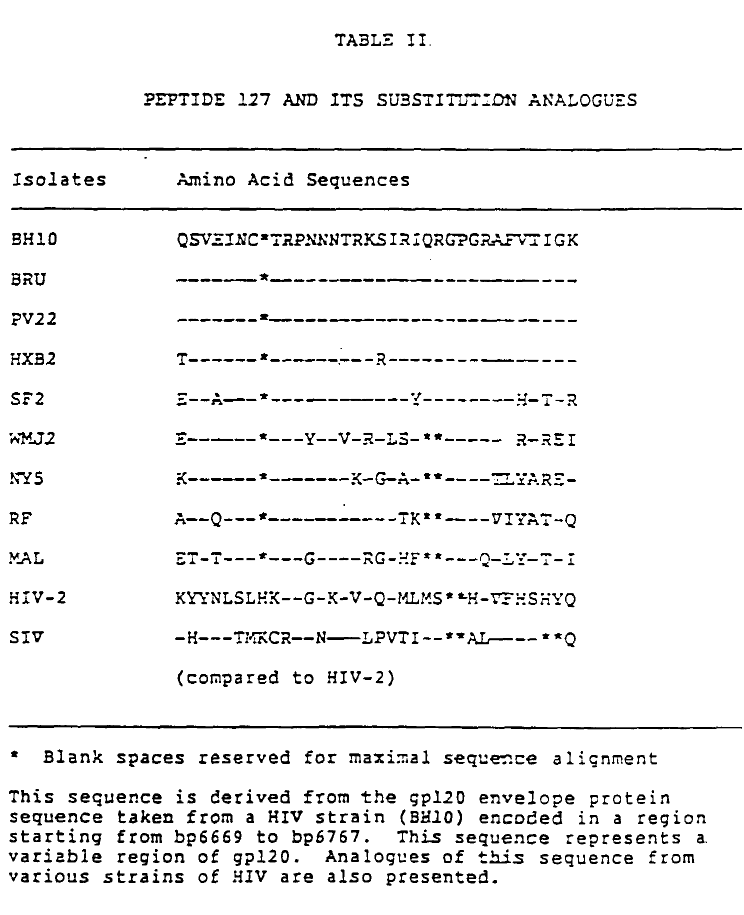

which separately or together provide equivalent sensitivity or I efficacy to the parent.

The peptides may be modified by introducing conservative or non-conservative substitutions in the peptides to more effectively mimic the differing epitopes of the different retroviral strains.

Further, to accommodate strain to strain variations among different isolates, adjustments for conservative substitutions and selection among the alternatives where non-conservative substitutions are involved, may be made. These peptides can be used individually or together for the detection of antibodies to the gp120 envelope protein in a physiological sample. Depending on the nature of the assay protocol, the peptides may be labelled or unlabelled, bound to a solid surface, polymeric or conjugated to a carrier or other compounds, or the like.

The polypeptides employed in the invention need not be identical to the specified sequence as long as the subject compounds are able to provide for immunological competition with gpl20 of at least one of the strains of the HIV retrovirus.

A specific peptide embodying the present invention is Peptide 127, a 33mer, which has the following amino acid sequence: QSVEINCTRPNNNTRKSIRIQRGPGRAFVTIGK-X, wherein X is OH or NH₂. This sequence corresponds to a small segment of the external envelope gp120 of HIV, or their analogues.

The peptides can be prepared in several ways. The peptides, because of their relatively small size, are easily synthesized by the solid phase strategy as developed by Merrifield (18) which is herein incorporated by reference. Various commercially available automatic peptide synthesizers with known protocols are used.

Compounds of this invention can also be prepared via recombinant DNA technology. In their preparation, a nucleotide sequence coding for the desired peptide or peptides in tandom arrangement representing the selected epitopes and their analogues, can be prepared using a conventional routine method for DNA synthesis. The synthesized oligonucleotides are ligated by a simple strategy previously described for the construction of a synthetic gene encoding such artificial protein containing the specified epitopes designated by the amino acid sequences of Peptide 127 or its analogues. The synthetic gene so constructed is transfected for optimal expression in Escherichia coli host cells.

The peptides of the present invention show a high degree of specific immunoreactivity with antibodies to HIV. A comparison of the results obtained for known serum samples by EIA using Peptide 127 as the solid phase immunoabsorbent with the results previously obtained for these known serum samples by diagnosis show that Peptide 127 is.highly specific in their immunoreactivity to antibodies to HIV. This shows that Peptide 127 defines highly reactive epitopes in the gp120 region of HIV.

Analysis of seropositivity with Peptide 127 by EIA on 30 well characterized sera with known neutralizing antibody titers revealed a high degree of correlation as calculated by Spearman Rank Correlation Coefficient, r=0.888, p<0.001 for Peptide 127. This means that a rapid EIA method using Peptide 127 can be used to replace the time consuming method of measuring serum neutralizing antibody titers.

HIV neutralizing antibody titers have previously been determined by reverse transcriptase assay which is very time consuming and produced highly variable results. For example, Prince et al. described a semi-automated microtiter assay which required culturing of serum specimens with HIV virus for 14 to 21 days and then repeatedly measuring reverse transcriptase activity of each sample in twelve replicates to obtain significant data. The method is extremely time consuming and variable. Further, Prince et al. showed that there was no correlation at all between neutralizing antibody titers obtained by this method and an ELISA method using deactivated virus as the immunosorbent (19).

In contrast, by using the peptides of the present invention a high degree of correlation was found. Thus, an EIA using the peptides of the present invention can be used to select plasma specimens containing high titers of neutralizing antibodies and to quantitatively determine HIV neutralizing antibody titers.

The peptides of the present invention are also useful as immunogens to elicit the production of high titers of anti-gp120 antibodies in mammals. This shows that the peptides are useful as immunogens and vaccines for the prevention of HIV infection.

The following examples employing Peptide 126 and Peptide 127 either as solid phase antigens in immunoassay or as immunogens in various forms to elicit specific and high titer anti-HIV gp120 antibodies.

### Example 1

### Detection of antibodies to HIV gp120 by EIA

Wells of 96 well plates were coated at 4°C over night with Peptide 126 or Peptide 127, prepared by standard solid phase peptide synthesis on a BIOSEARCH 9500 automated peptide synthesizer and cleaved from the resin by HF treatment. Each peptide was coated onto the wells at 1ug per well in 100uL 10mM NaHCO₃ buffer, pH 9.5. The wells were washed three times with phosphate-buffered saline (PBS) and then incubated with 250uL of 3% by weight gelatin in PBS at 37°C for 1 hour to block nonspecific protein binding sites, followed by three more washes with PBS containing 0.05% by volume Tween 20. The test sera from patients or normal blood donors were diluted 1:20 volume to volume with PBS containing 20% by volume normal goat serum, 1% by weight gelatin, and 0.05% by volume Tween 20. 200uL of the diluted sera were added to each well and allowed to react for 15 min at 37°C. The wells were then washed three times with 0.05% by volume Tween 20 in PBS in order to remove unbound antibodies. 100uL Horseradish peroxidase conjugated goat anti-human IgG at a 1:3000 dilution in 1% by volume normal goat serum, 0.05% by volume Tween 20 in PBS was added to each well at 37°C for 15 min as a second antibody tracer to bind with the antibody-antigen complex formed in positive wells.

The wells were washed five times with 0.05% by volume Tween 20 in PBS to remove unbound antibody and reacted with 100uL of the substrate mixture containing 0.04% by weight o-phenylenediamine (OPD) and 0.012% by volume hydrogen peroxide in sodium citrate buffer, pH 5.0. This substrate mixture was used to detect the peroxidase label by forming a color product. Reactions were stopped by the addition of 50uL of 1M H₂SO₄, and the color yield measured using an EIA reader at 492 nm. Absorbance readings greater than three times the average readings of 100 normal blood donor sera were taken as positive. The results are shown in Table III.

**Table III**

| DETECTION OF ANTIBODIES TO HIV BY EIA USING PEPTIDE 126 AND PEPTIDE 127 AS SOLID PHASE IMMUNOADSORBENT | | | |
|---|---|---|---|
| SUBJECT | NO. POSITIVE WITH gp41/p24 HIV-EIA SCREENING ASSAY*/NO. TESTED | NO. POSITIVE WITH GP120 PEPTIDE 126/NO. TESTED | NO. POSITIVE WITH GP120 PEPTIDE 127/NO. TESTED |
| 1. AIDS | 15/15 | 13/15 | 10/15 |
| | | | |
| 2. ARC | 8/8 | 4/8 | 6/8 |
| | | | |
| 3. HIV SEROPOS.INDIVISUALS | 17/17 | 16/17 | 12/17 |
| | | | |
| 4. LYMPHOID-LEUKEMIA MALIGNANCIES | 0/37 | 0/37 | 0/37 |
| | | | |
| 5. HTLV-I SEROPOS.INDIVIDUALS (ATL) | 0/50 | 0/50 | 0/50 |
| | | | |
| 6. AUTOIMMUNE DISEASES | 0/19 | 0/19 | 0/19 |
| | | | |
| 7. NORMAL BLOOD DONORS | 0/115 | 0/135 | 0/135 |
| Note: Sera from patients with AIDS, ARC, lymphoid-leukemia malignancies were kindly provided by Dr. Daniel M. Knowles of the New York University Medical School, Dr. Frederick P. Siegal of the Long Island Jewish Hospital along with detailed patient diagnosis and history. Sera from HTLV-I seropositive individuals including those with adult T-cell leukemia were provided by the Japanese Red Cross. Sera from patients with autoimmune diseases were provided by Dr. Nicholas Chiorazzi of the North Shore University Hospital, N.Y. along with their diagnosis and patient history. Human plasma from normal blood donors were obtained from the Hyland plasma center, I11. | | | |

| | | | |
|---|---|---|---|
| * Most of the HIV-EIA gp41/p24 screening assay of seropositive specimens gave an A₄₉₂ₙₘ of over 2.00. | | | |

The A₄₉₂ of EIA using Peptide 126 and Peptide 127 for various clinical data point are presented in Figure 1a and 1b.

As can be seen from the graphs, results obtained for EIAs employing Peptide 126 and Peptide 127 demonstrate a high degree of specificity in that all HIV seronegative individuals gave negative results with both assays, whereas those in the HIV seropositive groups showed varing degree of reactivities.

It is also observed that Peptide 126 based EIA gave higher absorbance and covers a broader HIV seropositive population (82.5%) than Peptide 127 based EIA (70%).

### Example 2

HIV-gp120 seropositivity by employing peptides 126 and 127 is unique to the two peptides described in this invention. A serum titration experiment was conducted using Peptide 126, Peptide 127, another peptide with an amino acid sequence of RPGGGDMRDNWRSELYKYKVVKIEPLGVAP (Peptide 065) and an 8mer peptide, with an amino acid sequence of ASTTTNYT (Peptide T). It is observed that "Peptide 065" and "Peptide T" have no immunoreactivity with sera from HIV seropositive individuals. See Table IV.

**TABLE IV**

| SPECIFIC IMMUNOREACTIVITY ASSOCIATED WITH HIV-GP120 PEPTIDES 126 and 127 | | | | |
|---|---|---|---|---|
| Dilution | A₄₉₂ₙₘ-EIA | | | |
| Serum No. N-9-13 | Peptide 126 | Peptide 127 | Peptide 065 | Peptide T |
| 1:20 | 2.64 +/- 0.02 | 2.52 +/- 0.01 | 0.04 +/- 0.01 | 0.02 +/- 0.00 |
| 1:40 | 2.47 +/- 0.01 | 2.04 +/- 0.01 | 0.01 +/- 0.01 | 0.03 +/- 0.00 |
| 1:80 | 2.14 +/- 0.02 | 1.41 +/- 0.01 | 0.06 +/- 0.02 | 0.02 +/- 0.00 |
| 1:160 | 1.63 +/- 0.02 | 0.93 +/- 0.01 | 0.05 +/- 0.02 | 0.00 +/- 0.00 |
| 1:320 | 1.04 +/- 0.01 | 0.55 +/- 0.00 | 0.03 +/- 0.00 | 0.02 +/- 0.00 |
| 1:640 | 0.62 +/- 0.00 | 0.32 +/- 0.00 | 0.04 +/- 0.01 | 0.03 +/- 0.00 |
| 1:1280 | 0.36 +/- 0.00 | 0.19 +/- 0.00 | 0.04 +/- 0.00 | 0.03 +/- 0.00 |

Serial titrations show that the immunoreactivity with Peptide 126 and Peptide 127 exhibited by HIV seropositive specimens are specific toward each epitope as shown in Figs. 2a and 2b. Only Peptide 127, but not Peptide 126 nor Peptide T, can inhibit the antibody immunoreactivity to Peptide 127. Similarly, Peptide 126, but not Peptide T and to a much less extent Peptide 127, can inhibit the immunoreactivity to Peptide 126.

### Example 3

A total of 30 sera, which have previously been carefully titered for their ability to neutralize HIV infection in cell culture using a method described by Prince (23), were tested at a 1:45 dilution by EIA employing (i) a gp41/p24 HIV-EIA kit, containing a mixture of three peptides known to be highly specific to antibodies to HIV: an 11mer peptide from the p24 region, a 19mer peptide and a 21mer peptide from the gp41 region (UBI, Hauppauge, N.Y.), (ii) Peptide 126, and (iii) Peptide 127 as the solid phase immunoadsorbent antigens. Results of this study are presented in Table V. Further data analysis indicated that there is a high degree of correlation between the neutralizing antibody titer of each serum and its immunoreactivity with either Peptide 126 or Peptide 127. See Fig. 3. The calculated Spearman Rank correlation coefficients between neutralizing antibody titer and EIA readings are r=0.949, p<0.001 for Peptide 126; and r=0.888, p<0.001 for Peptide 127.

Present methods employed to measure serum neutralizing antibody titers take about 14 to 21 days to perform and are subject to many experimental errors. This high degree of correlation between the rapid EIA method employing either Peptide 126 or Peptide 127 as the solid phase immunoadsorbent antigens and the known neutralizing antibody titers show that these rapid EIA methods using Peptide 126 or Peptide 127 can replace the prior cumbersome bioassay methods to determine the serum neutralizing antibody titer.

**Table V**

| RESULTS OF SERA TESTED BY NEUTRALIZATION ASSAY AND EIA EMPLOYING UBI-HIV-EIA KIT (p24/gp41) AND PEPTIDE 126 (gp 120) AND 127 (gp 120) AS IMMUNOADSORBENT ANTIGENS | | | | |
|---|---|---|---|---|
| Sample Code | Neutralization Titer | λ₄₉₂nm | | |
| | | Peptide 126 (gp 120) | Peptide 127 (gp 120) | Peptides-EIA (gp41/p24) |
| 1 | <1:4 | 0.05 | 0.09 | 0.07 |
| 2 | <1:4 | 0.05 | 0.09 | 0.06 |
| 3 | 1:128 | 2.69 | 1.71 | 2.88 |
| 4 | 1:16 | 0.50 | 0.14 | 1.13 |
| 5 | 1:32 | 0.68 | 0.12 | 1.68 |
| 6 | ≥1:512 | 2.67 | 1.89 | 2.92 |
| 7 | 1:6 | 0.04 | 0.05 | 0.07 |
| 8 | 1:8 | 0.20 | 0.07 | 2.12 |
| 11 | 1:100 | 2.56 | 1.10 | 2.62 |
| 12 | 1:8 | 0.20 | 0.22 | 0.13 |
| 14 | 1:4 | 0.06 | 0.08 | 0.07 |
| 15 | 1:64 | 0.21 | 0.13 | 1.00 |
| 16 | 1:4 | 0.05 | 0.07 | 0.09 |
| 17 | 1:4 | 0.05 | 0.07 | 0.06 |
| 18 | 1:512 | 1.75 | 2.05 | 1.50 |
| 19 | <1:4 | 0.06 | 0.07 | 0.10 |
| 21 | 1:64 | 0.14 | 1.19 | 1.71 |
| 22 | <1:4 | 0.05 | 0.07 | 0.07 |
| 23 | <1:4 | 0.05 | 0.13 | 0.06 |
| 25 | 1:8 | 0.09 | 0.11 | 0.13 |
| 26 | 1:64 | 2:61 | 2.06 | 2.52 |
| 27 | 1:4 | 0.09 | 0.07 | 0.27 |
| 28 | 1:4 | 0.07 | 0.08 | 0.12 |
| 29 | 1:64 | 0.08 | 0.08 | 0.19 |
| 32 | <1:4 | 0.04 | 0.08 | 0.07 |
| 33 | 1:256 | 1.07 | 1.53 | 0.54 |
| 34 | <1:4 | 0.03 | 0.08 | 0.07 |
| 36 | ≥1:512 | 2.62 | 0.98 | 0.69 |
| 37 | 1:256 | 2.45 | 1.16 | 2.17 |
| 38 | 1:100 | 2.54 | 1.73 | 2.59 |

### Example 4

A total of 13 sequential bleedings from two patients who underwent seroconversion were tested for their reactivity using the gp41/p24 HIV-EIA test kit (UBI, Hauppauge, N.Y.) and Peptide 126 and Peptide 127 as immunoadsorbents in an EIA method. The results show that the latency period for developing antibodies to gp120 Peptide 126 is longer than antibodies to the gp41/p24 epitopes and the latency period for developing antibodies to gp120 Peptide 127 was even longer, occurring much later in the course of infection. See Figs. 4a and 4b.

### Example 5

### Conjugation of Peptide 126 and Peptide 127 with Bovine Serum Albumin (BSA).

5 mg of BSA was dissolved in 5mL of PBS with gentle sonication. To the supernatant was added 4 mg of HF cleaved Peptide 126 or Peptide 127 followed by the addition of 40uL glutaldehyde (25%) to result in a final glutaldehyde concentration of 0.2%. The peptide-BSA and glutaldehyde mixture was incubated overnight at 4°C followed by an extensive dialysis with multiple changes (200X of its volume) of PBS. The dialysed peptide-BSA conjugate was then used together with either complete or incomplete Freund's adjuvant for administration to animals in an immunization program.

### Example 6

### Synthesis of Lysine Polymers of Peptides 126 and 127

The synthesis of an octameric form of Peptide 126 and Peptide 127 was initiated on a 4- methylbenzylhydrazine (MBHA) resin on which successive Boc-Lys (BOZ) were coupled to give a multiple antigen peptide system with eight reactive amino ends. The synthesis of Peptide 126 and Peptide 127 on this hexameric lysine resin with eight reactive amino ends was similar to the synthesis of the linear peptide using standard solid phase peptide synthesis strategy. The poly-L-lysine with eight units of Peptide 126 or Peptide 127 (see Tables VIa and VIb) were then liberated from the solid phase resin by HF cleavage procedure, extracted with acetic acid and lyopholized. The molecular weight as determined by single band SDS-PAGE of the octamers of Peptide 126 and Peptide 127 obtained, correlated well with the respective calculated molecular weights of 26,600 and 31,200. Analysis of the HF cleaved octameric Peptide 126 and Peptide 127 both gave broad peaks in C4 reverse phase HPLC.

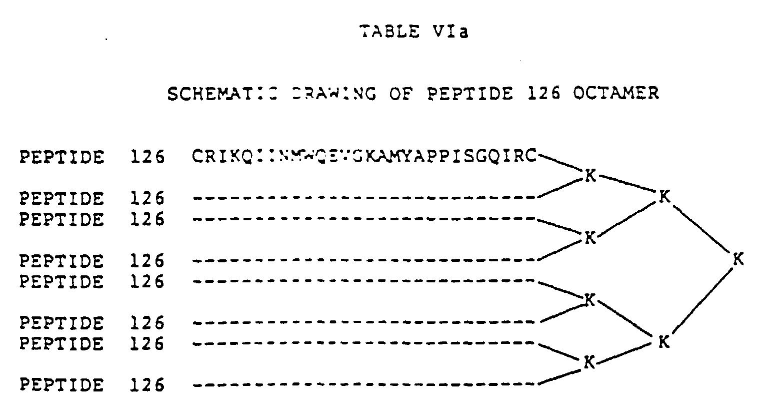

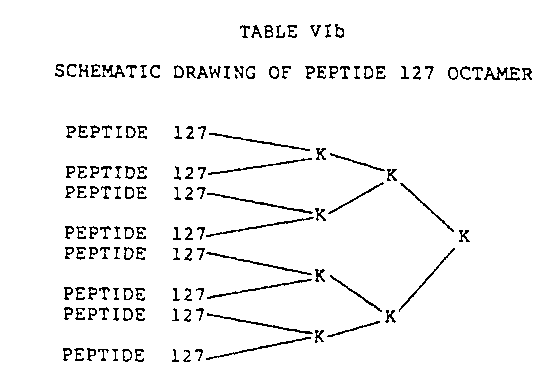

### Example 7

### Guinea Pig Immunization Protocol

Duncan Hartly closed colony random bred female guinea pigs, weighing 400-450 gms were used in all experiments. Each immunization was administered at the-described dosage by subcutaneous and intradermal injection in a volume of 1.0 mL to each member of two guinea pigs over multiple sites. A total of four groups were designed for immunization by: Peptide 126-BSA conjugate, Peptide 127-BSA conjugate, Peptide 126 octamer and Peptide 127 octamer. For the initial immunization, 100 ug peptide conjugate or octamer in 0.5 mL, was mixed with an equal volume of complete Freund's adjuvant and 1.0 mL was injected into each animal both subcutaneously and intradermally over multiple sites. After two to three weeks of rest, an identical dosage of each of the same immunogen was again injected as a booster shot both subcutaneously and intradermally into each animal except that incomplete Freund's adjuvant was used.

The animals were bled by heart puncture periodically to monitor their serum anti-gp120 titers and subsequent booster shots were given every two to six weeks. As shown in Figs. 6a, 6b, 6c and 6d, peptide 126-BSA, peptide 126 octamer, Peptide 127-BSA and Peptide 127 octomer all induced high titer (> 1 1000 - 1:10,000) antibodies to the corresponding : immunizing peptide after only one booster shot. High titers of antibodies cross reactive to the native HIV-gp120 protein were also produced as shown by Western Blot analysis. See Fig. 7.

The kinetics of the immune response generated by the specified immunization scheme indicated that effective, sustained level of the high serum antibody titers were elicited during the course of immunization particularly with the octameric form of the peptides. See Figs. 6a-d.

The results indicate that both Peptide 126 and Peptide 127 represent highly immunogenic epitopes present on the HIV gp120 protein. Sera obtained from guinea pigs previously immunized with the peptide conjugates or their polymeric forms induce high titers of antibodies to the immunizing peptide and are found to be highly cross reactive to the native gp120 as demonstrated by the Western Blot analysis. See Fig 7.

It is quite remarkable that synthetic peptides representing minute fractions of the external envelope protein of a large virus can be manipulated to mimic the antigenic/immunogenic sites to an extent that neutralizing or protective antibodies can be elicited by immunizing with these well-defined peptides and quantitative EIAs employing these synthetic peptides as the solid phase immunoadsorbent can be used to detect natural antibodies to gp120 present in HIV infected individuals to an extent that their neutralizing ability can be predicted by the signal obtained from these EIA results. Based on this high degree of correlation with neutralizing antibody titers it is expected that these peptides, their analogues and mixtures in either the conjugated or polymeric forms, will be useful as candidates for synthetic vaccines.

## Claims

1. A peptide composition characterised in that it exhibits specific immunoreactivity to antibodies to HIV and elicits the production of neutralizing antibodies to HIV, which is selected from:
(i) QSVEINCRTRPNNNTRKSIRIQRGPGRAFVTIGK (Peptide 127);
(ii) an analogue thereof having an amino acid sequence derived from a strain/isolate of HIV in a region corresponding to the peptide;
(iii) a conjugate thereof with a carrier;
(iv) a polymer thereof; and
(v) a mixture of the peptides with an analogue, conjugate or polymer thereof.

2. A peptide composition as claimed in claim 1 wherein it comprises a conjugate of Peptide 127 and a protein having a molecular weight of at least 5,000.

3. A peptide composition as claimed in claim 2 wherein the protein is bovine serum albumin.

4. A peptide composition as claimed in claim 1 wherein it comprises:

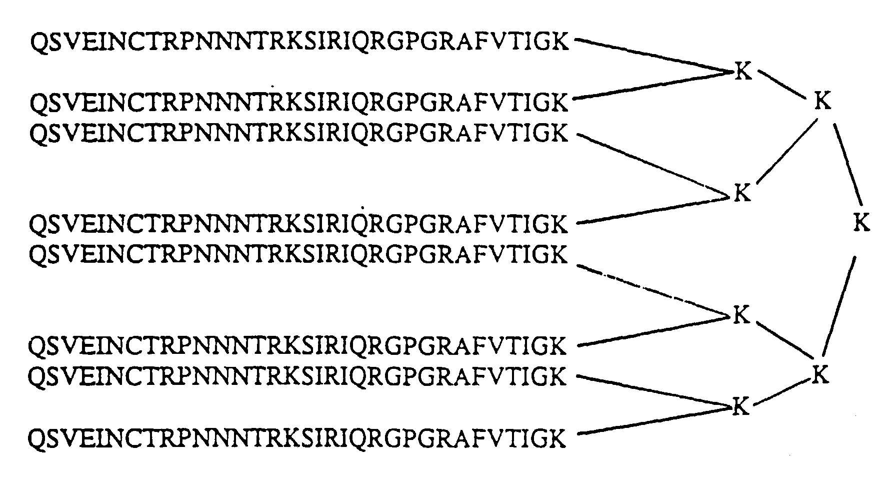

5. A method for detecting the presence of antibodies to HIV and the presence of neutralizing antibodies to HIV-gp120 in an immunoassay characterised in that it comprises using as the immunosorbent a peptide composition as claimed in claim 1.

6. An analogue of peptide-127 characterised in that it is selected from:

(i) TSVEINCTRPNNNTRKRIRIQRGPGRAFVTIGK (Peptide-127HXB2)
(ii) ESVAINCTRPNNNTRKSIRYQRGPGRAFHTTGR (Peptide-127SP2)
(iii) ESVEINCTRVYRNLSKRIQRGPGRAFRTREI (Peptide-127WMJ2)
(iv) KSVEINCTRPNNNTKKGIAIGPGRTLYAREK (Peptide-127NY5)
(v) ASVQINCTRPNNNTRKSITKGPGRVIYATGQ (Peptide-127RF)
(vi) ETVTINCTRPGNNTRRGIHFGPGQALYTTGI (Peptide-127MAL)
(vii) KYYNLSLHKRPGNKTVKQIMLMSHRVFHSHYQ (Peptide-127HIV-2).

7. Neutralizing antibodies for AIDS virus characterised in that they are elicited by the use of a peptide composition as claimed in any of claims 1 to 4 or 6.

## Patentansprüche

1. Eine Peptidzusammensetzung, dadurch gekennzeichnet, daß sie spezifische Immunreaktivität auf Antikörper gegenüber HIV aufweist und die Produktion von neutralisierenden Antikörpern gegenüber HIIV hervorruft, die ausgewählt ist aus:
(i) QSVEINCRTRPNNNTRKSIRIQRGPGRAFVTIGK (Peptid 127);
(ii) einerm Analog davon mit einer Aminosäuresequenz, die von einem Stamm/ Isolat von HIV stammt in einer Region entsprechend dem Peptid;
(iii) einem Konjugat davon mit einem Träger;
(iv) einem Polymer davon und
(v) einem Gemisch der Peptide mit einem Analog, Konjugat oder Polymer davon.

2. Eine Peptidzusammensetzung gemäß Anspruch 1, worin sie ein Konjugat von Peptid 127 und ein Protein mit einem Molekulargewicht von mindestens 5000 enthält.

3. Eine Peptidzusammensetzung gemäß Anspruch 2, worin das Protein Rinderserumalbumin ist.

4. Eine Peptidzusammensetzung gemäß Anspruch 1, welche enthält:

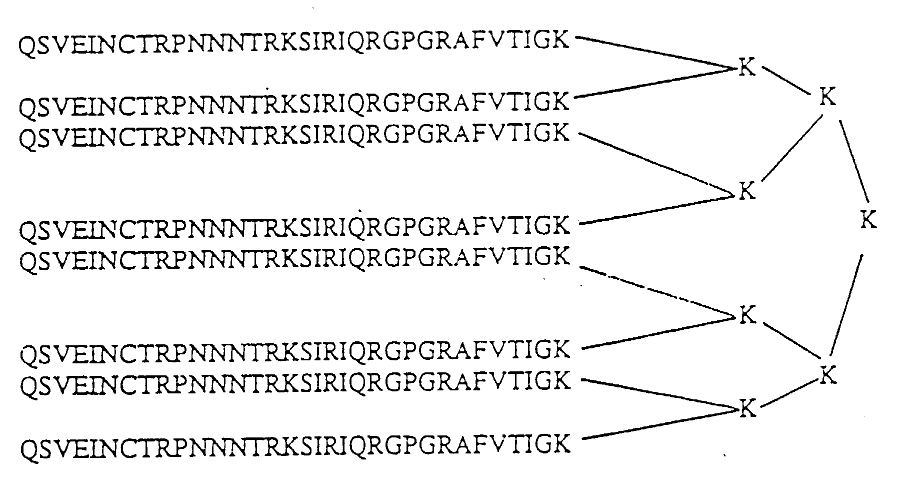

5. Eine Methode zur Ermittlung der Gegenwart von Antikörpern gegenüber HIV und der Gegenwart von neutralisierenden Antikörpern gegenüber HIV-gp-120 in einem Immunassay, dadurch gekennzeichnet, daß sie als das Immunosorbent eine Peptidzusammensetzung gemäß Anspruch 1 verwendet.

6. Ein Analog von Peptid-127, dadurch gekennzeichnet, daß es ausgewählt ist aus:

(i) TSVEINCTRPNNNTRKRIRIQRGPGRAFVTIGK (Peptide-127HXB2)
(ii) ESVAINCTRPNNNTRKSIRYQRGPGRAFHTTGR (Peptide-127SP2)
(iii) ESVEINCTRVYRNLSKRIQRGPGRAFRTREI (Peptide-127WMJ2)
(iv) KSVEINCTRPNNNTKKGIAIGPGRTLYAREK (Peptide-127NYS)
(v) ASVQINCTRPNNNTRKSITKGPGRVIYATGQ (Peptide-127RF)
(vi) ETVTINCTRPGNNTRRGIHFGPGQALYTTGI (Peptide-127MAL)
(vii) KYYNLSLHKRPGNKTVKQIMLMSHRVFHSHYQ (Peptide-127HIV-2).

7. Neutralisierende Antikörper für das AIDS-Virus, dadurch gekennzeichnet, daß sie hervorgerufen werden durch die Verwendung einer Peptidzusammensetzung gemäß einem der Ansprüche 1 bis 4 oder 6.

## Revendications

1. Une composition à base de peptides, caractérisée en ce qu'elle présente une immuno réactivité spécifique aux anticorps de l'HIV et permet d'obtenir la production d'anticorps neutralisants de l'HIV, choisie à partir de :
(i) 
QSVEINCRTRPNNNTRKSIRIQRGPGRAFVTIGK (Peptide
127) ;
(ii) un analogue de ce dernier présentant une séquence d'acide aminé dérivée d'une souche/isolat de l'HIV dans une zone correspondant au peptide ;
(iii) un conjugué de celui-ci avec un support ;
(iv) un polymère de celui-ci ; et
(v) un mélange des peptides avec un analogue, un conjugué ou un polymère de celui-ci.

2. Une composition à base de peptides telle que revendiquée à la revendication 1, comprenant un conjugué de Peptide 127 et une protéine présentant un poids moléculaire d'au moins 5000.

3. Une composition à base de peptides telle que revendiquée à la revendication 2, dans laquelle la protéine est de l'albumine de sérum bovin.

4. Une composition à base de peptides telle que revendiquée à la revendication 1, comprenant :

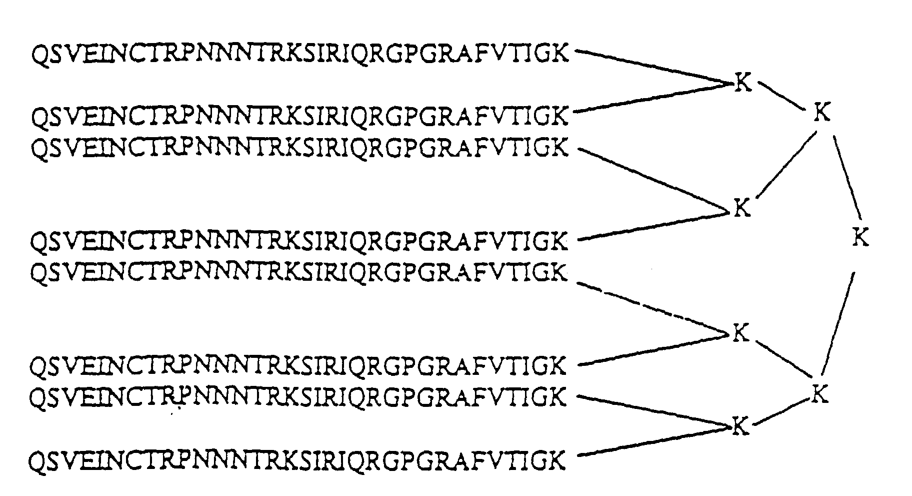
QSVEINCTRPNNNTRKSIRIQRGPGRAFVTIGK
K
QSVEINCTRPNNNTRKSIRIQRGPGRAFVTIGK
K
QSVEINCTRPNNNTRKSIRIQRGPGRAFVTIGK
K
K
QSVEINCTRPNNNTRKSIRIQRGPGRAFVTIGK
QSVEINCTRPNNNTRKSIRIQRGPGRAFVTIGK
K
QSVEINCTRPNNNTRKSIRIQRGPGRAFVTIGK
QSVEINCTRPNNNTRKSIRIQRGPGRAFVTIGK
K
K
QSVEINCTRPNNNTRKSIRIQRGPGRAFVTIGK

5. Un procédé pour détecter la présence d'anticorps de l'HIV et la présence d'anticorps neutralisants de l'HIV-gp120 dans un immuno essai, caractérisé en ce qu'il comprend le fait d'utiliser, en tant qu'immuno sorbant, une composition à base de peptides telle que revendiquée à la revendication 1.

6. Un analogue de peptide 127 caractérisé en ce qu'il est choisi à partir de :

(i) TSVEINCTRPNNNTRKRIRIQRGPGRAFVTIGK (Peptide-127HXB2)

(ii) ESVAINCTRPNNNTRKSIRYQRGPGRAFHTTGR (Peptide-127SF2)

(iii) ESVEINCTRVYRNLSKRIQRGPGRAFRTREI (Peptide-127WMJ2)

(iv) KSVEINCTRPNNNTKKGIAIGPGRTLYAREK (Peptide-127NY5)

(v) ASVQINCTRPNNNTRKSITKGPGRVIYATGQ (Peptide-127RF)

(vi) ETVTINCTRPGNNTRRGIHFGPGQALYTTGI (Peptide-127MAL)

(vii) KYYNLSLHKRPGNKTVKQIMLMSHRVFHSHYQ (Peptide-127HIV-2).

7. Anticorps neutralisants pour le virus du SIDA caractérisés en ce qu'ils sont obtenus par l'utilisation d'une composition à base de peptides telle que revendiquée dans une quelconque des revendications 1 à 4 ou 6.
